# EUROPÄISCHE PATENTANMELDUNG

(11) **EP 0 824 095 A1**
(43) Veröffentlichungstag der Anmeldung: **18.02.1998**
(21) Anmeldenummer: 97113644.5
(22) Anmeldetag: 07.08.1997
(51) Int. Cl.: C07C 45/50, C07C 45/80, C07F 9/02

(54) **Verfahren zur Abtrennung von Phosphinoxiden und Alklarylphosphinen aus Reaktionsgemischen einer homogenen Hydroformylierung**

(30) Priorität: 13.08.1996 DE 19632530
(71) Anmelder: HOECHST AKTIENGESELLSCHAFT, 65926 Frankfurt am Main (DE)
(72) Erfinder: Bahrmann, Helmut, Dr., 46499 Hamminkeln (DE); Müller, Thomas, 46535 Dinslaken (DE)

(57) **Zusammenfassung**

Die Erfindung betrifft ein Verfahren zur Abtrennung von Phosphinoxiden und Alkylarylphosphinen aus dem organischen Reaktionsgemisch einer homogenen Hydroformylierung, die unter Verwendung eines Katalysatorsystems durchgeführt wird, welches metallorganische Komplexverbindungen sowie im molaren Überschuß Ammoniumsalze aromatischer Phosphine als Liganden enthält. Das Verfahren ist dadurch gekennzeichnet, daß man das organische Reaktionsgemisch einer extraktiven Behandlung mit einer 0,001 - 0,5 Gew.-%igen Alkali- oder Erdalkalihydroxidlösung unterwirft und anschließend die die Phosphinoxide und Alkylarylphosphine enthaltende wäßrige Phase abtrennt.

Durch Anwendung der erfindungsgemäßen Extraktion gelingt es, die Konzentration der Phosphinoxide und Alkylarylphosphine in dem Hydroformylierungsgemisch deutlich zu senken. Das Verfahren eignet sich insbesondere für kontinuierlich betriebene Hydroformylierungen.

## Beschreibung

Die vorliegende Erfindung betrifft ein Verfahren zur Abtrennung von Phosphinoxiden und Alkylarylphosphinen aus Reaktionsgemischen einer homogenen Hydroformylierung.

Die technisch in großem Umfang durchgeführte Hydroformylierung von Olefinen erfolgt zunehmend in Gegenwart von Katalysatorsystemen auf der Basis von Rhodium-Komplexverbindungen, die tertiäre Phosphine oder Phosphite als Liganden enthalten. Da die Liganden in der Regel im Überschuß vorliegen, besteht das Katalysatorsystem aus metallorganischen Komplexverbindungen und zusätzlichem reinem Ligand. Entsprechend der Löslichkeit dieser Katalysatorsysteme in organischen Medien erfolgt die Hydroformylierung in homogener Phase.

Zur Abtrennung des Reaktionsproduktes und Wiedergewinnung des im Reaktionsgemisch homogen gelösten Katalysatorsystems destilliert man das Reaktionsprodukt im allgemeinen aus dem Reaktionsgemisch ab. Dies ist aber wegen der thermischen Empfindlichkeit der gebildeten Aldehyde nur bei der Hydroformylierung von niederen Olefinen mit bis zu etwa 8 Kohlenstoffatomen im Molekül möglich. Bei der Hydroformylierung langkettiger Olefine oder olefinischer Verbindungen mit funktionellen Gruppen werden thermisch empfindliche Produkte oder Produkte mit hohem Siedepunkt gebildet, die sich destillativ nicht mehr zufriedenstellend vom Katalysator abtrennen lassen: Die thermische Belastung des Destillationsgutes führt durch Dickölbildung zu erheblichen Verlusten an Wertprodukt sowie durch Zersetzung der Komplexverbindungen zu Verlusten an Katalysator. Hierdurch wird die wirtschaftliche Attraktivität des Verfahrens entscheidend verringert.

Um die Abtrennung des Katalysatorsystems auf thermischem Weg zu vermeiden, wurden verschiedene Verfahrensalternativen entwickelt. Aus EP-A-0 216 375 ist ein Verfahren zur Herstellung von Aldehyden durch Umsetzung von Olefinen mit Wasserstoff und Kohlenmonoxid in homogener Phase in Gegenwart eines Rhodium sowie aromatische Phosphine im molaren Überschuß als Liganden enthaltenden Katalysatorsystems bekannt, bei dem als aromatische Phosphine in organischen Medien lösliche und in Wasser unlösliche Ammoniumsalze sulfonierter oder carboxylierter Triarylphosphine eingesetzt werden.

Die Ammoniumionen besitzen die Formeln (NR₂H₂)⁺ und/oder (NR₃H)⁺, wobei R für Alkylreste mit 4 bis 12 Kohlenstoffatomen oder für Aryl- oder Cycloalkylreste mit 6 bis 12 Kohlenstoffatomen steht.

Zur Abtrennung des Katalysatorsystems vom Reaktionsprodukt behandelt man in diesem Fall das Hydroformylierungsgemisch zunächst mit einer Base, z.B. Alkali- oder Erdalkalihydroxid-Lösungen. Hierbei werden aus den (NR₂H₂)⁺- bzw. (NR₃H)⁺-Salzen die entsprechenden sekundären oder tertiären Amine freigesetzt, und gleichzeitig wird ein wasserlösliches Alkali- oder Erdalkalisalz des sulfonierten oder carboxylierten Triarylphosphins gebildet, welches dadurch in die wäßrige Phase gelangt und über eine Extraktion zusammen mit dem komplex am Phosphor gebundenen Rhodium von der das Hydroformylierungsprodukt enthaltenden organischen Phase abgetrennt werden kann.

Eine weitere Möglichkeit, das Katalysatorsystem einer homogenen Hydroformylierung aus dem Reaktionsgemisch abzutrennen, ist aus der deutschen Patentanmeldung mit dem Aktenzeichen 196 19 527.6 bekannt. Das eingesetzte Katalysatorsystem enthält wasserunlösliche Rhodium-Komplexverbindungen sowie als Liganden Ammoniumsalze sulfonierter, carboxylierter oder phosphonierter aromatischer Diphosphine und wird nach der Hydroformylierung durch Membranfiltration an einer semipermeablen Polyaramidmembran aus dem Reaktionsgemisch abgetrennt.

Im Verlauf derartiger homogener Hydroformylierungsreaktionen kommt es jedoch zu Neben- und Abbaureaktionen am Katalysatorsystem, und dabei insbesondere an den Liganden. Durch Oxidation des Phosphor(III) in den jeweiligen Ammoniumsalzen der aromatischen Phosphine entstehen Phosphinoxide. Ferner bilden sich im Laufe der Hydroformylierung aus den aromatischen Phosphinen Alkylarylphosphine, indem Arylreste der aromatischen Phosphine gegen Alkylgruppen ausgetauscht werden, wobei sich diese Alkylgruppen von dem Olefin ableiten, das hydroformyliert wird. Sowohl die Phosphinoxide als auch die Alkylarylphosphine liegen dabei in Form ihrer Ammoniumsalze vor. Während die Phosphinoxide keine Komplexbildung mehr mit dem verwendeten Metall, insbesondere Rhodium, eingehen können und damit zu einer allmählichen Verarmung des Katalysatorsystems an Ligand führen, sind die Alkylarylphosphine weiterhin zur Bildung von Komplexen befähigt. Diese Komplexe sind jedoch katalytisch nicht oder nur wenig aktiv sind und wirken somit aktivitätsmindernd auf die Hydroformylierung. Diese Phosphinoxid- sowie Alkylarylphosphinbildung stellt vor allem dann ein Problem dar, wenn die Hydroformylierung kontinuierlich durchgeführt wird oder dieselbe Katalysatorlösung mehrfach eingesetzt wird und sich die Phosphinoxide sowie Alkylarylphosphine somit über einen längeren Zeitraum immer weiter anreichern können.

Aufgabe der vorliegenden Erfindung war es daher, ein Verfahren zur Abtrennung der katalytisch unwirksamen Phosphinoxide und desaktivierend wirkenden Alkylarylphosphinen aus dem Reaktionsgemisch einer homogenen Hydroformylierung zur Verfügung zu stellen, mit dem die Konzentration der Phosphinoxide und Alkylarylphosphine im Reaktionsgemisch der Hydroformylierung verringert werden kann.

Gelöst wird diese Aufgabe durch ein Verfahren zur Abtrennung von Phosphinoxiden und Alkylarylphosphinen aus dem organischen Reaktionsgemisch einer homogenen Hydroformylierung, die unter Verwendung eines Katalysatorsystems durchgeführt wird, welches metallorganische Komplexverbindungen sowie im molaren Überschuß Ammmoniumsalze aromatischer Phosphine als Liganden enthält, dadurch gekennzeichnet, daß man das organische Reaktionsgemisch einer extraktiven Behandlung mit einer 0,001 - 0,5 Gew.-%igen wäßrigen Alkali- oder Erdalkalihydroxidlösung unterwirft und anschließend die die Phosphinoxide und Alkylarylphosphine enthaltende wäßrige Phase abtrennt.

Bei der zur extraktiven Behandlung eingesetzten Alkali- oder Erdalkalihydroxid-Lösung handelt es sich vorzugsweise um Natronlauge oder Kalilauge. Die Konzentration dieser wäßrigen Alkali- oder Erdalkalihydroxid-Lösung beträgt 0,001 - 0,5 Gew.-%, bevorzugt 0,01 - 0,05 Gew.-%.

Das der extraktiven Behandlung zu unterziehende organische Reaktionsgemisch stammt aus einer homogenen Hydroformylierung zur Herstellung von Aldehyden durch Umsetzung von olefinischen Verbindungen mit Wasserstoff und Kohlenmonoxid.

Das bei der Hydroformylierung eingesetzte Katalysatorsystem enthält metallorganische Komplexverbindungen sowie im molaren Überschuß Ammoniumsalze aromatischer Phosphine als Liganden. Bei diesen Ammoniumsalzen aromatischer Phosphine handelt es sich erfindungsgemäß um Ammoniumsalze aromatischer Mono- oder Diphosphine.

Als Ammoniumsalze aromatischer Monophosphine werden bevorzugt Alkyl- und/oder Arylammoniumsalze sulfonierter oder carboxylierter Triarylphosphine der allgemeinen Formel I 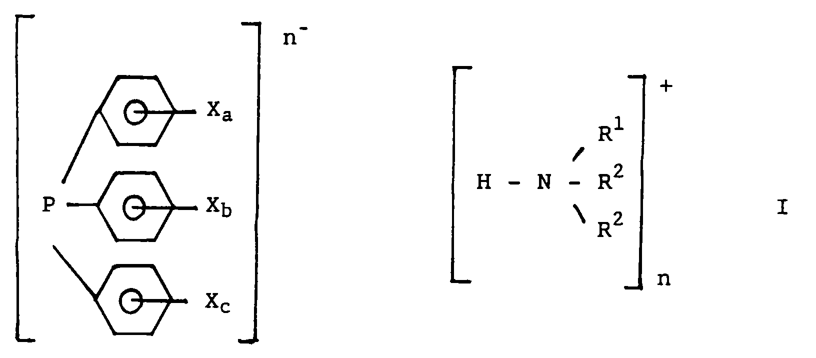 eingesetzt, worin X einen Sulfonat- oder Carboxylatrest bedeutet, a, b und c gleich oder verschieden und O oder 1 sind, wobei mindestens einer der Parameter a, b oder c gleich 1 sein muß, n gleich 1, 2 oder 3 ist, R¹ und R² gleich oder verschieden und C₄ - C₃₀-Alkylreste oder C₆ - C₁₀-Aryl- oder Cycloalkylreste bedeuten und R¹ auch Wasserstoff sein kann.

Die homogene Hydroformylierung von Olefinen unter Verwendung von Katalysatorsystemen, die als Liganden Verbindungen der Formel I enthalten, ist in einer am gleichen Tag eingereichten deutschen Patentanmeldung sowie in der EP-A-0 216 375 beschrieben.

Als weitere Vertreter der Ammoniumsalze aromatischer Monophosphine werden Verbindungen der allgemeinen Formel II eingesetzt, 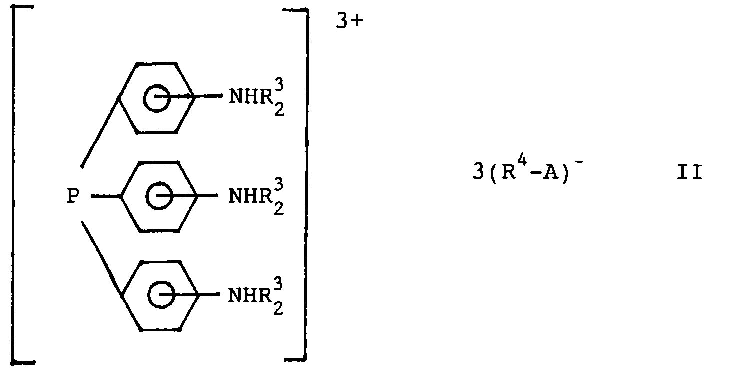 worin R³ für Wasserstoff oder einen C₁ - C₁₂-Alkylrest, R⁴ für einen C₁ - C₂₅-Alkylrest oder einen C₆ - C₁₀-Arylrest und A für einen Sulfonat-(SO₃⁻)- oder Carboxylat(COO⁻)-rest oder Phosphonat-Rest (R-PO₃²⁻) steht.

Als Vertreter der Ammoniumsalze aromatischer Diphosphine werden bevorzugt Verbindungen der Formel III 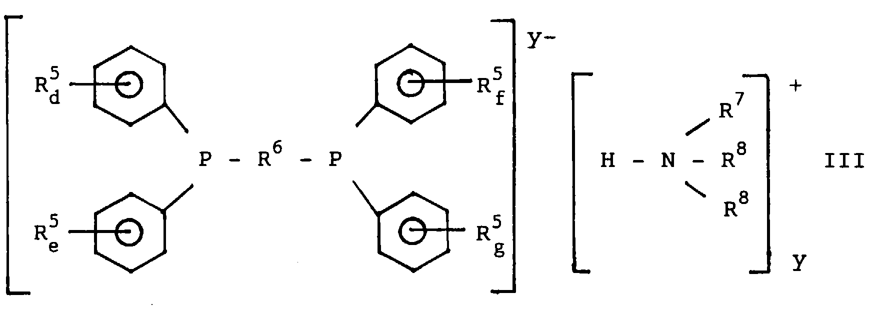 worin R⁵ einen Carboxylat-(COO⁻), Sulfonat-(SO₃⁻), Phosphonat-(PO₃²⁻) oder 2-Aminoethanbisphosphonat-Rest ⁅NH-CH₂-CH(PO₃²⁻)₂] darstellt, R⁶ für einen geradkettigen Alkylenrest mit 1 - 8 Kohlenstoffatomen, einen Sauerstoff enthaltenden Alkylenrest mit 2 - 6 Kohlenstoffatomen, einen Cycloalkylenrest mit 3 bis 10 Kohlenstoffatomen oder einen Rest der Formeln IV, V, VI oder VII 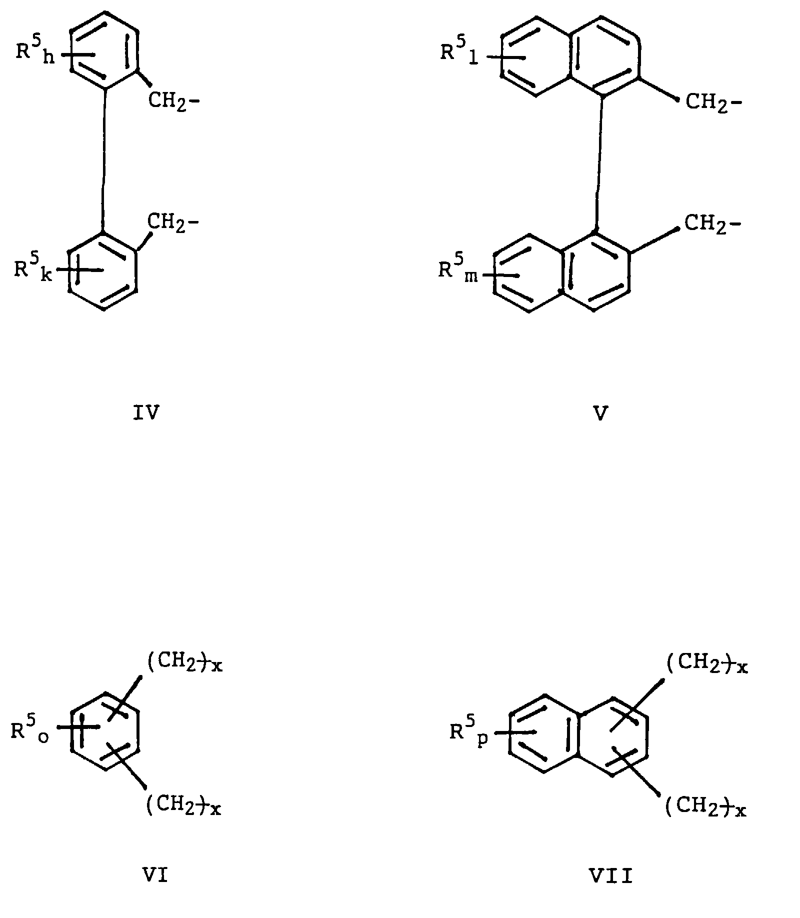 steht, d, e, f, g, h, k, l, m, o und p gleich oder verschieden und 0 oder 1 sind, wobei mindestens einer der Parameter d, e, f, g, h, k, l, m, o oder p gleich 1 sein muß, y gleich der Summe der Parameter d, e, f, g, h, k, l, m, o und p ist, x gleich oder verschieden und 0 oder 1 ist, R⁷ und R⁸ gleich oder verschieden sind und für C₄ - C₂₆-Alkyl-, substituierte oder unsubstituierte C₆ - C₁₀-Aryl- oder C₆ - C₁₀-Cycloalkylreste oder einen Benzylrest stehen und R⁷ auch Wasserstoff bedeuten kann.

Die homogene Hydroformylierung von Olefinen unter Verwendung von Katalysatorsystemen, die als Liganden Verbindungen der Formel III enthalten, ist in der deutschen Patentanmeldung mit dem Aktenzeichen 196 19 527.6 beschrieben.

Als Ammoniumsalze aromatischer Diphosphine haben sich ferner Verbindungen der allgemeinen Formel VIII bewährt, 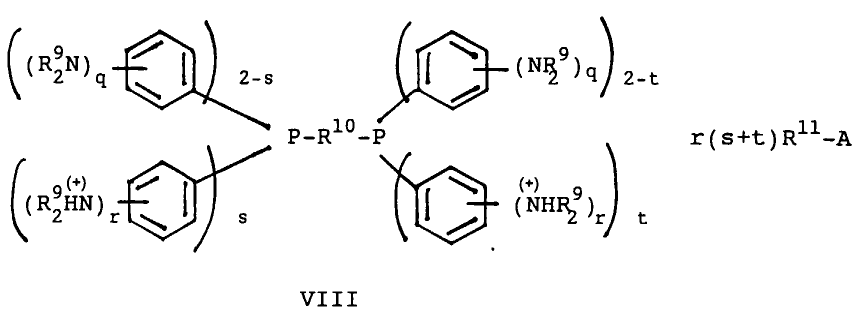 worin R⁹ für Wasserstoff oder einen C₁ - C₁₂-Alkylrest, R¹⁰ für einen geradkettigen C₁ - C₈-Alkylenrest, einen Sauerstoff enthaltenden C₂ - C₄-Alkylenrest, einen C₃ - C₁₀-Cycloalkylenrest oder einen Rest der Formel IX oder X steht, 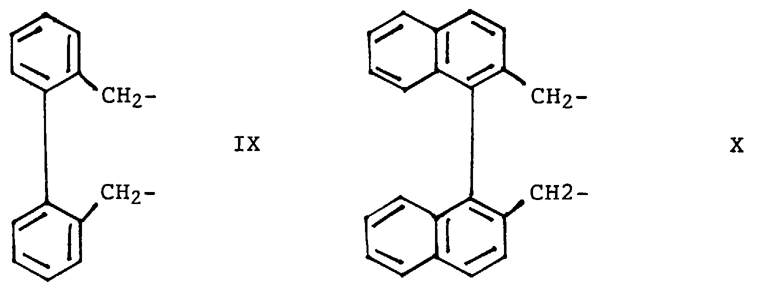 R¹¹ einen C₁ - C₂₅-Alkylrest oder einen C₆ - C₁₀-Arylrest darstellt, A für einen Carboxylat-(COO⁻) oder Sulfonat-(SO₃⁻)-Rest steht und q = 0, r = 1, s = 1 und t = 1 ist, oder q = 1, r = 1, s = (1 oder 2) und t = (1 oder 2) ist, oder, falls R¹⁰ einen Rest der Formel IX oder X darstellt, q = 1, r = 0, s = (0 oder 1) und t = (0 oder 1) ist.

Die homogene Hydroformylierung von Olefinen unter Verwendung von Katalysatorsystemen, die als Liganden Verbindungen der Formel VIII enthalten, ist in der deutschen Patentanmeldung mit dem Aktenzeichen 196 09 337.6 beschrieben.

Im Rahmen der homogenen Hydroformylierung ist es nicht erforderlich, die Ammoniumsalze der aromatischen Phosphine gemäß den Formeln I, II, III und VIII im Katalysatorsystem als einheitliche Verbindungen einzusetzen. Es können z.B. auch unterschiedliche Sulfonierungsstufen der Phosphine und/oder Sulfonatgemische mit verschiedenen Ammonium-Kationen umgesetzt werden.

In der Hydroformylierung werden olefinisch ungesättigte Verbindungen mit 2 bis 30 Kohlenstoffatomen eingesetzt, die eine oder mehrere Doppelbindungen besitzen können. Geeignet sind substituierte oder unsubstituierte Alkene mit 6 bis 30 Kohlenstoffatomen, substituierte oder unsubstituierte Diene mit 4 bis 10 Kohlenstoffatomen, substituierte oder unsubstituierte Cycloalkene oder Dicycloalkene mit 5 bis 12 Kohlenstoffatomen im Ringsystem, Ester einer ungesättigten Carbonsäure mit 3 bis 20 Kohlenstoffatomen und eines aliphatischen Alkohols mit 1 bis 18 Kohlenstoffatomen, Ester einer gesättigten Carbonsäure mit 2 bis 20 Kohlenstoffatomen und eines ungesättigten Alkohols mit 2 bis 18 Kohlenstoffatomen, ungesättigte Alkohole oder Ether mit jeweils 3 bis 20 Kohlenstoffatomen oder araliphatische Olefine mit 8 bis 20 Kohlenstoffatomen.

Bei den substituierten oder unsubstituierten Alkenen mit 6 bis 30 Kohlenstoffatomen kann es sich um geradkettige oder verzweigte Alkene mit endständiger oder innenständiger Lage der Doppelbindung handeln. Bevorzugt sind geradkettige Olefine mit 6 bis 18 Kohlenstoffatomen wie n-Hexen-1, n-Hepten-1, n-Octen-1, n-Nonen-1, n-Decen-1, n-Undecen-1, n-Dodecen-1, n-Octadecen-1 und acyclische Terpene. Geeignet sind auch verzweigte Alkene wie Diisobutylen (2,4,4-Trimethylpenten-1), Tripropylen, Tetrapropylen und Dimersol (Dibutylen).

Bevorzugte Beispiele für unsubstituierte Diene mit 4 bis 10 Kohlenstoffatomen sind 1,3-Butadien, 1,5-Hexadien und 1,9-Decadien.

Beispiele für substituierte und unsubstituierte Cycloalkene oder Dicycloalkene mit 5 bis 12 Kohlenstoffatomen im Ringsystem sind Cyclohexen, Cycloocten, Cyclooctadien, Dicyclopentadien und cyclische Terpene wie Limonen, Pinen, Camphoren und Bisabolen. Bevorzugt ist Dicyclopentadien.

Beispielhaft für araliphatische Olefine mit 8 bis 20 Kohlenstoffatomen steht Styrol.

Als Beispiele für Ester einer ungesättigten Carbonsäure mit 3 bis 20 Kohlenstoffatomen und eines aliphatischen Alkohols mit 1 bis 18 Kohlenstoffatomen seien die Acrylsäureester und die Methacrylsäureester mit 1 - 18 Kohlenstoffatomen in der Alkohol-Komponente genannt.

Zu den Estern einer gesättigten Carbonsäure mit 2 - 20 Kohlenstoffatomen und eines ungesättigten Alkohols mit 2 - 18 Kohlenstoffatomen gehören die Vinyl- und Allylester mit 2 - 20 Kohlenstoffatomen in der Carbonsäurekomponente, beispielsweise Vinylacetat.

Zu den ungesättigten Alkoholen und Ethern zählen beispielsweise die Allylalkohole und Vinylether.

Die im Verlauf der Hydroformylierung durch Oxidation des Phosphor(III) in den Ammoniumsalzen der aromatischen Phosphine entstehenden Phosphinoxide sowie die ebenfalls gebildeten Alkylarylphosphine liegen als Ammoniumsalze vor. Diese Ammoniumsalze werden bei Zugabe der wäßrigen Alkali- oder Erdalkalihydroxidlösung in die entsprechenden Alkali- oder Erdalkalisalze überführt, welche wasserlöslich sind und daher in die wäßrige Phase gehen. Von wesentlicher Bedeutung ist hierbei, daß die zur Extraktion eingesetzte Alkali- oder Erdalkalihydroxid-Lösung eine Konzentration im Bereich von 0,001 - 0,5 Gew.-% besitzt. Nur durch Einhaltung dieses Bereichs kann verhindert werden, daß auch die Liganden selbst, d.h. die Ammoniumsalze der aromatischen Phosphine, in höherem Maß in die wäßrige Phase übergehen und ferner basisch katalysierte Folgereaktionen, wie die Aldolisierung der gebildeten Aldehyde, unterbleiben. Es hat sich ferner bewährt, die Extraktion bei einem möglichst niedrigen pH-Wert des organischen Reaktionsgemisches durchzuführen. Besonders bevorzugt ist dabei ein pH-Wert von 2,5 - 4,0, insbesondere 2,8 - 3,6.

Bei der Extraktion des Reaktionsgemisches aus einer homogenen Hydroformylierung wird neben den Alkali- oder Erdalkalisalzen der Phosphinoxide bzw. Alkylarylphosphine in entsprechender Menge auch Amin freigesetzt, welches sich in der Katalysatorlösung anreichert, jedoch keinerlei negativen Einfluß auf diese ausübt. Durch Zugabe einer entsprechenden Menge frischer Phosphine können die jeweiligen Alkyl- und/oder Arylammoniumsalze der allgemeinen Formeln I, II, III und VIII wieder regeneriert werden. Die Zugabe der Phosphine kann entweder gleichzeitig mit der Alkali- oder Erdalkalihydroxidlösung oder aber erst nach der Extraktion erfolgen.

Das erfindungsgemäße Verfahren kann in der folgenden Weise durchgeführt werden.

Das bei der homogenen Hydroformylierung entstehende organische Reaktionsgemisch wird aus dem Reaktor entnommen. Es enthält die hergestellten Aldehyde, nicht umgesetzte Olefine, das Katalysatorsystem aus metallorganischer Komplexverbindung und den Ammoniumsalzen der aromatischen Phosphine sowie die abzutrennenden Phosphinoxide, Alkylarylphosphine und gegebenenfalls andere Abbau- und Nebenprodukte. Dieses Reaktionsgemisch wird ein- oder mehrfach entspannt, um nicht umgesetztes Synthesegas als Abgas zu entfernen. Es hat sich hierbei bewährt, die zur Extraktion einzusetzenden Alkali- oder Erdalkalihydroxidlösungen dem Reaktionsgemisch der Hydroformylierung vor der Entspannung hinzuzufügen, um so den bei der Entspannung auftretenden Vermischungseffekt auszunutzen. Die Abtrennung der wäßrigen, die Phosphinoxide und Alkylarylphosphine enthaltenden Phase erfolgt im Anschluß an die Entspannung mittels eines herkömmlichen Phasentrenners. Dieser Extraktionsschritt kann mehrfach hintereinander durchgeführt werden. Es ist auch möglich, die Extraktion der Phosphinoxide und Alkylarylphosphine erst im Anschluß an die Entspannung in bekannten Extraktionsapparaturen, wie Mixer-Settler-Batterien, durchzuführen. Die Extraktion kann bei erhöhter Temperatur von 50 - 90°C oder auch bei Raumtemperatur durchgeführt werden. Das nach der Abtrennung der wäßrigen Phase zurückbleibende organische Reaktionsgemisch wird anschließend bevorzugt einer Membranfiltration unterworfen, wobei das Katalysatorsystem vom Hydroformylierungsprodukt abgetrennt wird.

Das erfindungsgemäße Verfahren ermöglicht es, die Konzentration der Phosphinoxide und der Alkylarylphosphine in dem organischen Reaktionsgemisch einer homogenen Hydroformylierung deutlich zu reduzieren. Bevorzugt findet das Verfahren Anwendung auf Reaktionsgemische von kontinuierlich betriebenen homogenen Hydroformylierungen. Bereits durch eine einfache Extraktion kann dabei eine Menge an Phosphinoxiden und Alkylarylphosphinen aus dem Reaktionsgemisch der Hydroformylierung abgetrennt werden, die weit über der in jedem Verfahrensdurchgang der kontinuierlichen Reaktion gebildeten Menge dieser Abbauprodukte liegt. Durch mehrfache Extraktion können die Phosphinoxide und Alkyldiarylphosphine in noch höherem Maße abgetrennt werden. In den meisten Fällen reicht es jedoch bereits aus, lediglich einen Teilstrom des Reaktionsgemisches der Hydroformylierung der oben beschriebenen Extraktion zu unterwerfen, oder aber die Extraktion nur zeitweise in Betrieb zu nehmen.

### Beispiele

Folgende Abkürzungen werden verwendet:
- TPPOTS:: Triphenylphosphinoxidtrisulfonat
- TPPODS:: Triphenylphosphinoxiddisulfonat
- PDSPP :: Propyldisulfophenylphosphin
- TPPTS :: Triphenylphosphintrisulfonat
- TPPDS :: Triphenylphosphindisulfonat
- BSNS :: Benzolsulfonsäurenatriumsalz

### Beispiel 1

Ein Reaktionsgemisch aus der Hydroformylierung von Propylen mit einem Katalysatorsystem aus Rhodium-Komplexverbindungen und dem Distearylammoniumsalz des TPPTS als Ligand enthält 46,98 mmol Phosphor(III)/kg und 10,81 mmol Phosphor(V)/kg.

148 g eines solchen Reaktionsgemisches (6,86 mmol Phosphor(III) und 1,58 mmol Phosphor(V)) werden bei 70°C mit 10 ml 0,05 Gew.-%iger Natronlauge 15 Minuten extrahiert. Nach 10 Minuten Phasentrennung wird die wäßrige Phase (9,28 g) abgetrennt und HPLC-analytisch analysiert. Es werden die in Tabelle 1 dargestellten Substanzmengen bestimmt.

**Tabelle 1**

| Extrahierte Substanz | mmol in der wäßrigen Phase | % vom Einsatz |
|---|---|---|
| TPPOTS | 0,052 | 3,29 |
| TPPODS | 0,004 | 0,25 |
| PDSPP | 0,4 · 10⁻⁴ | 0,03 |
| TPPTS | 8,7 · 10⁻⁴ | 0,01 |
| TPPDS | 6,2 · 10⁻⁴ | 0,01 |

Aus Tabelle 1 ist zu erkennen, daß bereits durch einen einzigen Extraktionsvorgang 3,29 % des im Reaktionsgemisch vorhandenen TPPOTS und 0,25 % des TPPODS entfernt werden können. Dies ist bereits deutlich mehr, als größenordnungsmäßig im Rahmen eines Durchgangs der kontinuierlichen Hydroformylierung neu gebildet wird. Durch mehrfache Wiederholung des Extraktionsschrittes kann die extrahierte Menge der Phosphinoxide entsprechend vervielfacht werden.

### Beispiel 2

Eine Hydroformylierung von Dicyclopentadien in Toluol mit einem Katalysatorsystem aus Rhodium-Komplexverbindungen und dem Distearylammoniumsalz des TPPTS als Ligand im molaren Überschuß (21 ppm Rhodium, Phosphor : Rhodium-Verhältnis = 50, Toluol-Anteil 60 Gew.-%) liefert ein Reaktionsgemisch mit einer Phosphor(III)-Konzentration von 11,9 mmol/kg und einer Phosphor(V)-Konzentration von 11,9 mmol/kg. 207 g dieses Reaktionsgemisches werden bei 70°C für 60 Minuten der Extraktion mit einer 0,04 %igen Natronlauge unterworfen. Nach 5 Minuten Phasentrennung wird die wäßrige Phase abgetrennt. Die Phosphor(V)-Konzentrationen für TPPDS und TPPOTS werden mittels HPLC analysiert.

Die Phosphor(III)-Konzentration wird titrimetrisch ermittelt. Es werden die in Tabelle 2 dargestellten extrahierten Substanzmengen bestimmt.

Die abgetrennte organische Phase wird mit 1,53 ml einer 5 %igen Natronlauge versetzt und anschließend erneut mit 206,1 g einer 0,04 %igen Natronlauge unter analogen Bedingungen wie zuvor beschrieben, extrahiert.

Die wäßrige Phase wird nach Phasentrennung, wie beschrieben analysiert.

Die organische Phase wird zwei weitere Male, ebenfalls wie beschrieben extrahiert. Die Ergebnisse sind in Tabelle 2 zusammengefaßt.

**Tabelle 2**

| Zahl der Extraktionsstufen | Extrahierte Substanzen [Gew.-%, bezogen auf die in der zur Extraktion eingesetzte Reaktionsmischung vorhandenen Mengen der jeweiligen Substanz] | | | |
|---|---|---|---|---|
| | TPPTS | TPPODS | TPPOTS | BSNS |
| 1 | - | 17 | 31 | 21 |
| 2 | 1.2 | 26 | 56 | 29 |
| 3 | 3.3 | 40 | 80 | 29 |
| 4 | 10.7 | 62 | 98 | 36 |

## Patentansprüche

1. Verfahren zur Abtrennung von Phosphinoxiden und Alkylarylphosphinen aus dem organischen Reaktionsgemisch einer homogenen Hydroformylierung, die unter Verwendung eines Katalysatorsystems durchgeführt wird, welches metallorganische Komplexverbindungen sowie im molaren Überschuß Ammoniumsalze aromatischer Phosphine als Liganden enthält, dadurch gekennzeichnet, daß man das organische Reaktionsgemisch einer extraktiven Behandlung mit einer 0,001 - 0,5 Gew.-%igen Alkali- oder Erdalkalihydroxidlösung unterwirft und anschließend die die Phosphinoxide und Alkylarylphosphine enthaltende wäßrige Phase abtrennt.

2. Verfahren nach Anspruch 1, dadurch gekennzeichnet, daß als Alkalihydroxidlösung Natronlauge oder Kalilauge verwendet wird.

3. Verfahren nach Anspruch 1 oder 2, dadurch gekennzeichnet, daß die Konzentration der Alkali- oder Erdalkalihydroxidlösung 0,01 - 0,05 Gew.-% beträgt.

4. Verfahren nach einem oder mehreren der Ansprüche 1 - 3, dadurch gekennzeichnet, daß es sich bei den Ammoniumsalzen der aromatischen Phosphine um Ammoniumsalze aromatischer Mono- oder Diphosphine handelt.

5. Verfahren nach Anspruch 4, dadurch gekennzeichnet, daß als Ammoniumsalze aromatischer Monophosphine Alkyl- und/oder Arylammoniumsalze sulfonierter oder carboxylierter Triarylphosphine der allgemeinen Formel I verwendet werden 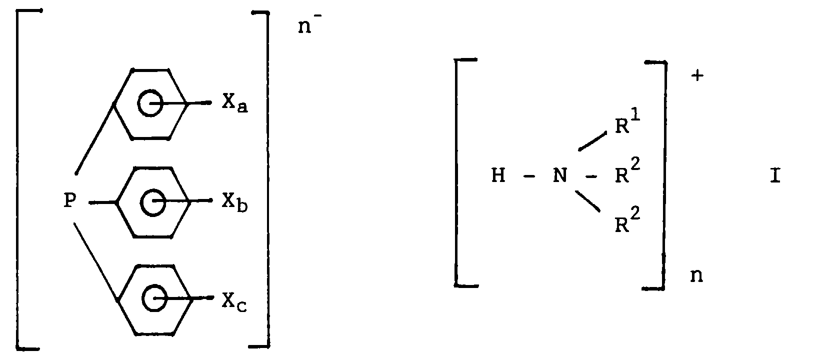 worin X einen Sulfonat-(SO₃⁻) oder Carboxylat-(COO⁻)-Rest bedeutet, a, b und c gleich oder verschieden und O oder 1 sind, wobei mindestens einer der Parameter a, b oder c gleich 1 sein muß, n gleich 1, 2 oder 3 ist, R¹ und R² gleich oder verschieden und C₄ - C₃₀-Alkylreste oder C₆ - C₁₀-Aryl- oder Cycloalkylreste bedeuten und R¹ auch Wasserstoff sein kann.

6. Verfahren nach Anspruch 4, dadurch gekennzeichnet, daß als Ammoniumsalze aromatischer Monophosphine Verbindungen der allgemeinen Formel II eingesetzt werden, 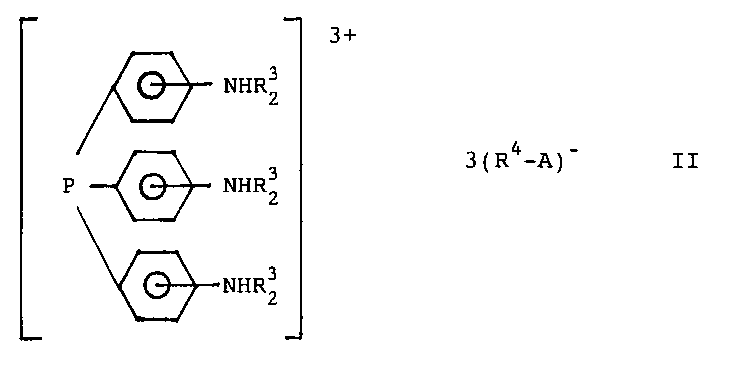 worin R³ für H oder einen C₁ - C₁₂-Alkylrest, R⁴ für einen C₁ - C₂₅-Alkylrest oder einen C₆ - C₁₀-Arylrest und A für einen Sulfonat-(SO₃⁻)- oder Carboxylat-(COO⁻)-rest oder Phosphonat-Rest (R-PO₃²⁻) steht.

7. Verfahren nach Anspruch 4, dadurch gekennzeichnet, daß als Ammoniumsalze aromatischer Diphosphine Verbindungen der Formel III eingesetzt werden, 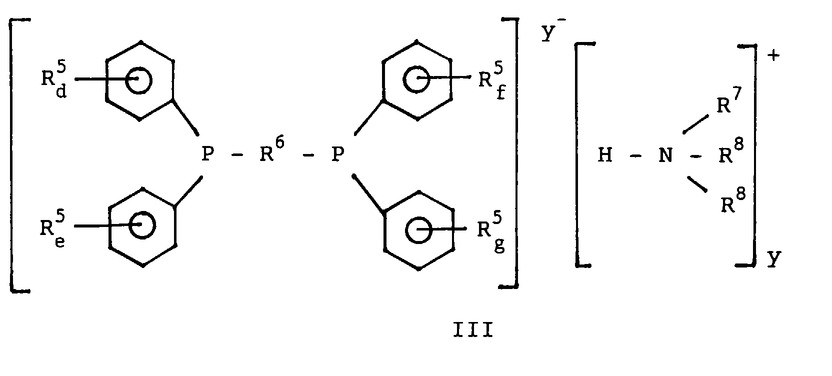 worin R⁵ einen Carboxylat-(COO⁻), Sulfonat-(SO₃⁻), Phosphonat-(PO₃²⁻) oder 2-Aminoethanbisphosphonat-Rest ⁅NH-CH₂-CH(PO₃²⁻)₂] darstellt, R⁶ für einen geradkettigen Alkylenrest mit 1 - 8 Kohlenstoffatomen, einen Sauerstoff enthaltenden Alkylenrest mit 2 - 6 Kohlenstoffatomen, einen Cycloalkylenrest mit 3 bis 10 Kohlenstoffatomen oder einen Rest der Formeln IV, V, VI oder VII 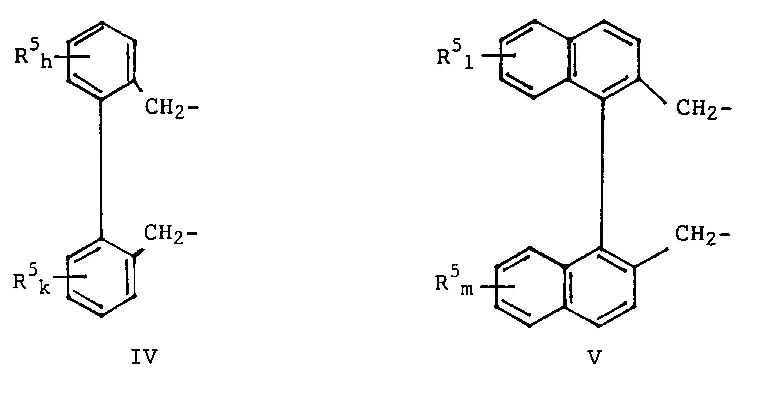 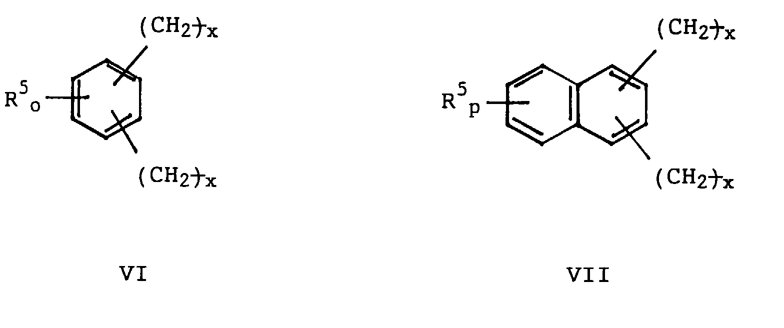 steht, d, e, f, g, h, k, l, m, o und p gleich oder verschieden und 0 oder 1 sind, wobei mindestens einer der Parameter d, e, f, g, h, k, l, m, o oder p gleich 1 sein muß, y gleich der Summe der Parameter d, e, f, g, h, k, l, m, o und p ist, x gleich oder verschieden und 0 oder 1 ist, R⁷ und R⁸ gleich oder verschieden sind und für C₄ - C₂₆-Alkyl-, substituierte oder unsubstituierte C₆ - C₁₀-Aryl- oder C₆ - C₁₀-Cycloalkylreste oder einen Benzylrest stehen und R⁷ auch Wasserstoff bedeuten kann.

8. Verfahren nach Anspruch 4, dadurch gekennzeichnet, daß als Ammoniumsalze aromatischer Diphosphine Verbindungen der allgemeinen Formel VIII eingesetzt werden, 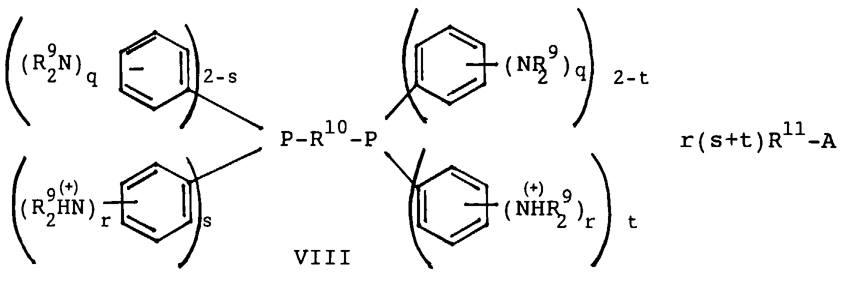 worin R⁹ für H oder einen C₁ - C₁₂-Alkylrest, R¹⁰ für einen geradkettigen C₁ - C₈-Alkylenrest, einen Sauerstoff enthaltenden C₂ - C₄-Alkylenrest, einen Rest der Formel IX oder X 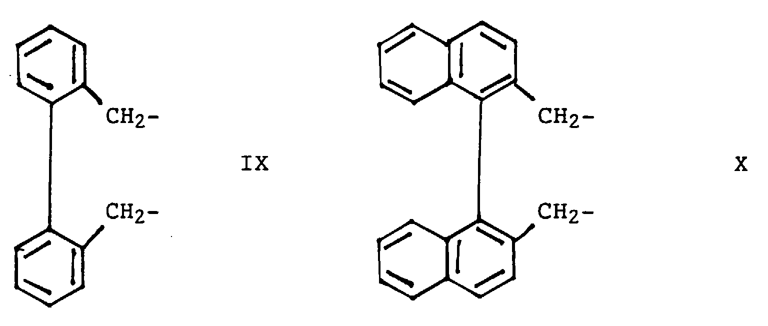 oder einen C₃ - C₁₀-Cycloalkylenrest, R¹¹ für einen C₁ - C₂₅-Alkylrest oder einen C₆ - C₁₀-Arylrest steht, A einen Carboxylat-(COO⁻) oder Sulfonat-(SO₃)-Rest darstellt und q = 0, r = 1, s = 1 und t = 1 ist, oder q = 1, r = 1, s = (1 oder 2) und t = (1 oder 2) ist, oder, falls R¹⁰ einen Rest der Formel IX oder X darstellt, q = 1, r = 0, s = (0 oder 1) und t = (0 oder 1) ist.

9. Verfahren nach einem oder mehreren der Ansprüche 1 - 8, dadurch gekennzeichnet, daß man das nach Abtrennung der wäßrigen Phase zurückbleibende organische Reaktionsgemisch einer Membranfiltration unterwirft.
